# EUROPEAN PATENT APPLICATION

(11) **EP 3 309 467 A1**
(43) Date of publication of application: **18.04.2018**
(21) Application number: 16807802.0
(22) Date of filing: 09.06.2016
(51) Int. Cl.: F24F 3/16, B60H 3/00, C25B 1/04

(54) **COOLING APPARATUS FOR KILLING FUNGI ON DEW CONDENSATION PART BY MEANS OF HYDROGEN GENERATED BY ELECTROLYZING WATER CONDENSED AT DEW CONDENSATION PART OF COOLING APPARATUS**

(30) Priority: 09.06.2015 KR 20150081039
(71) Applicant: Tak, Seung Ho, Seoul 06747 (KR)
(72) Inventor: Tak, Seung Ho, Seoul 06747 (KR)
(74) Representative: Weickmann & Weickmann PartmbB
(86) International application number: PCT/KR2016/006097
(87) International publication number: WO 2016/200162

(57) **Abstract**

Provided are: a vehicle cooling apparatus and a residential and office cooling apparatus, which are improved so as to prevent offensive odors and respiratory diseases by killing fungi and bacteria inside of a cooling part by means of hydrogen generated by electrolyzing the water condensed at the cooling part of the cooling apparatus; or a cooling apparatus and an air conditioning apparatus having a health-beneficial hydrogen generation device added thereto in which bacteria do not propagate since hydrogen is mixed and supplied to the interior air by means of a heat pump such that air quality is improved and the quality-improved air is supplied.

## Description

### [CROSS REFERENCE TO RELATED APPLICATIONS]

This application claims the benefit under 35 USC 119(a) of PCT Application No. PCT/KR2016/006097, filed on June 9, 2016, which claims the benefit of Korean Patent Application No. 10-2015-0081039 filed on June 9, 2015, in the Korean Intellectual Property Office, the entire disclosure of which is incorporated herein by reference for all purposes.

### [Technical Field]

When a cooling apparatus used in a household and office and a vehicle cooling apparatus are operated, moisture in air undergoes condensation (3) and is frozen at a cooling part (hereinafter, referred to as an evaporator), and then the frozen moisture is melted when the cooling apparatus stops operating, and thus fungi and bacteria live at a periphery (8) of the evaporator occurs. Thus, a universally used wing-type blower (2-1) performs forceful circulation (9) of the fungi in the air, which leads to odors spreading and air causing respiratory diseases and skin problems being provided in an indoor space. However, a fundamental problem of fungi and bacteria propagation has not been addressed even when an antibacterial plastic and an antibacterial filter are adopted at main regions, fungi inside the evaporator are killed by a plasma ion device being additionally installed or an ultraviolet ray sterilizer being mounted, or regular cleaning is performed.

### [Background Art]

When a cooling apparatus for a household, an office, a food and pharmaceutical factory, and a vehicle is operated, moisture undergoes condensation 3 and is frozen at an evaporator. Then, when the cooling apparatus stops operating and is left at room temperature, fungi live in a region 8 of the evaporator, which leads to odors spreading and many health problems such as respiratory diseases being caused, however there is no measure to be taken to rectify such a situation.

In order to address the problems, an antibacterial plastic is adopted at main regions of a cooling apparatus, an antibacterial air filter is applied to the main regions, a plasma negative ion generator is installed at a region of an evaporator, an ultraviolet (UV) ray sterilizer is mounted at the region of the evaporator, and regular cleaning is performed using chemicals, however, because the cooling apparatus is used under a high humidity and high temperature climate condition during the summer season, for example, in a vehicle, it is inevitable that condensation and freezing occur at the region of the evaporator. In addition, when an ignition of a vehicle is turned off, fungi and bacteria start to propagate at a condensation region. Although a fungicide is sprayed to remove odors caused by the fungi or a filter is regularly replaced, generation of fungi is not fundamentally prevented, and such a problem has been neglected.

In particular, a henhouse, a pigsty, and a cattle shed at which animals, such as chickens, pigs, and cows, are kept are contaminated by bacteria, and thus mass death due to an infectious disease such as avian influenza, foot-and-mouth disease, and the like, have regularly broken out and odors from excretion are generated. However, when animals are fed on electrolyzed water and inhale air mixed with hydrogen, an infectious disease caused by bacteria and odors can be prevented because hydrogen exhibits high sterilizing power.

### [Disclosure]

### [Technical Problem]

When a cooling apparatus is operated in a high humidity and temperature indoor space or vehicle, moisture in the enclosed indoor space condenses and freezes on an evaporator of the cooling apparatus, and then, when the cooling apparatus stops operating, a temperature inside the evaporator increases so that pathogenic bacteria such as fungi and legionella are generated, spread odors, and cause respiratory and skin diseases.

In order to address the problems, water condensed in a condensation part of the cooling apparatus is electrolyzed to generate hydrogen to kill fungi in the condensation part of the cooling apparatus by sterilization, and therefore, odors are prevented and respiratory and skin diseases caused by bacteria such as legionella are prevented from occurring. Furthermore, an improvement allows pleasant and healthy cooling with indoor air mixed with hydrogen to be provided.

In addition to an air conditioner (cooling apparatus), during repetitive operations in which a freezer and a refrigerator are automatically turned on or off to maintain a temperature after having water condensed and frozen thereon, fungi and bacteria propagate and deteriorate food stored in the refrigerator for a long period of time.

When water condensed and frozen in a refrigerator and a freezer is electrolyzed to generate hydrogen inside the refrigerator, fungi and bacteria are killed by hydrogen ions by sterilization and are unable to propagate, which improves storage of food inside the refrigerator and the freezer for a long period of time.

Specifically, an air conditioner mixed with hydrogen may address a problem which is not solved with an ultraviolet (UV) lamp or an air purifier that is installed to prevent fungi or bacteria from propagating inside a food factory or a pharmaceutical factory requiring adherence to the Hazard Analysis and Critical Control Points (HACCP) standard.

In the case of a vehicle cooling apparatus, when high temperature and humidity air inside the vehicle is cooled in the summer season, water condensation 3 and freezing occurs at a cooling part, and then, when the vehicle is turned off, an indoor temperature of the vehicle increases and fungi propagates at the cooling part, which leads to odors spreading and respiratory and skin diseases being caused. An electrolysis electrode 10 is installed in a water holder 5 under an evaporator of the vehicle cooling apparatus or a water bucket 13 is installed under a funneled drain pipe 6 from the outside of the vehicle, the electrolysis electrode 10 is installed inside the water bucket 13, and a 12 volt or 24 volt power 25 which is completely isolated from a power of the vehicle is converted into a direct current 27 using a flyback power supply so that currents 32 and 33 flow to the electrolysis electrode 10 so that water 14 is electrolyzed to generate hydrogen. When the hydrogen is provided to the evaporator 1, fungi and bacteria 8 are killed so that odors and respiratory and skin diseases are prevented. In addition, when air mixed with hydrogen is provided to an inner space of the vehicle, a driver feels less fatigue and drives the vehicle in a refreshing and healthy manner.

Because a negative electrode of a commercialized battery used in most vehicles is used as a ground for a chassis of a vehicle, power which is completely isolated from the power of the vehicle is generated and then electrolysis is performed to prevent an electrolysis reaction from occurring between a voltage applied to a platinum-plated titanium electrode for electrolysis and the negative electrode which is the ground of the chassis.

### [Technical Solution]

Water has a chemical formula of "H₂O", that is, as two hydrogen atoms and one oxygen atom are combined to form water, hydrogen and oxygen can be produced via electrolysis of water. However, even though distilled water without an electrolyte is also the same water, a current does not flow in distilled water, and therefore it is impossible to electrolyze distilled water.

However, when moisture in air condenses and freezes at an evaporator, metal is finely dissolved in water so that the water is not distilled water and becomes electrolyzable water in which an electrolyte is mixed. In addition, the water condenses and freezes in a state in which it includes an electrolyte due to dust in the air, which is not distilled water, and therefore electrolyzing the water is enough to generate hydrogen for sterilization.

However, because oxygen as well as hydrogen is generated while electrolyzing water, ozone, which is harmful to a human body, is produced when the oxygen is dissolved in water. According to a hydrogen generator cited in Korean Patent Application No. 10-2015-0103257 filed on July 21, 2015, when a porous ceramic is installed near an electrode to prevent the production of ozone and hydrogen is generated to kill fungi using negative hydrogen ions by sterilization, problems of fungi and bacteria propagation caused by condensation occurring at a cooling part of a cooling apparatus, a freezer, a refrigerator, and a dehumidifier may be addressed.

### [Advantageous Effects]

When a cooling apparatus is applied to a transportation method such as a vehicle, an aircraft, and a ship by which a person travels, a building such as a residence, a food factory or a pharmaceutical factory requiring adherence to the Hazard Analysis and Critical Control Points (HACCP) standard, a hospital, a residential facility for the elderly and the infirm, and general indoor spaces, it is not possible to prevent condensation of moisture in air at a cooling part, and the condensed and frozen moisture becomes a breeding ground for fungi and bacteria, which leads to bad smells being generated and respiratory and skin diseases being caused.

When hydrogen is generated by electrolyzing water condensed and frozen at a cooling part, is transferred to an entrance of a low noise blower which does not include any wings and produce any primary noise, and passes through an evaporator, 99% or more of fungi and bacteria can be killed to stop fungi and bacteria from propagating. In addition, pure cold air mixed with hydrogen is provided in the air so that invisible fungi produced in a corner due to moisture in indoor air are killed. Furthermore, a person can healthily respire because active oxygen of a human body is combined with hydrogen such that antioxidant effects are exhibited when the person inhales hydrogen.

In addition, when indoor air of a cage in which chickens, pigs, and cows are kept, such as a cattle shed, a henhouse, a pigsty, and the like, a plant factory, and a greenhouse to which a good agriculture practice (GAP) process is applied is sterilized using hydrogen electrolyzed from water, a hydrogen dehumidifying and humidifying apparatus, a hydrogen air conditioner, and a hydrogen cooling apparatus using a technique of spraying the hydrogen electrolyzed from water into the air, an infectious disease, avian influenza, and foot-and-mouth disease of animals and plants can be prevented, and odors caused by excretion can be prevented, and therefore effects in which animals are healthily raised can be exhibited.

Furthermore, when indoor air of a food processing and manufacturing factory, a pharmaceutical factory, and a food distribution and sales store that follow the HACCP standard and GMP process is sterilized using hydrogen electrolyzed from water, a hydrogen dehumidifying and humidifying apparatus, a hydrogen air conditioner, and a hydrogen cooling apparatus, and then processed food, manufactured food, and medicine are processed, manufactured, distributed, and sold, spoilage of the food and medicine is prevented. Therefore, an effect in which shelf life is extended as much as possible is exhibited using a method other than a storage and distribution temperature method.

Furthermore, when it is necessary to produce drinking water by sterilizing water such as rainwater, ground water, and condensed water, killing intestinal bacteria or pathogenic bacteria with hydrogen is the fastest, safest and most economical way to produce drinking water. Therefore, producing drinking water by sterilizing water with hydrogen produced via electrolyzing water is effective.

### [Description of Drawings]

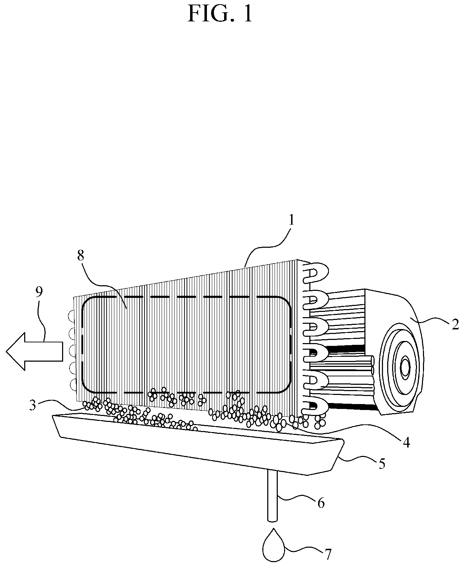

FIG. 1 illustrates an example of propagation of fungi caused by condensation and freezing of water at an evaporator of a conventional cooling apparatus.

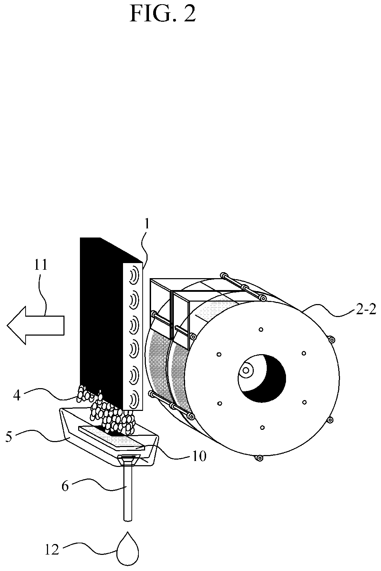

FIG. 2 illustrates an example in which an electrolysis hydrogen generator is applied to a water holder of an evaporator of a cooling apparatus.

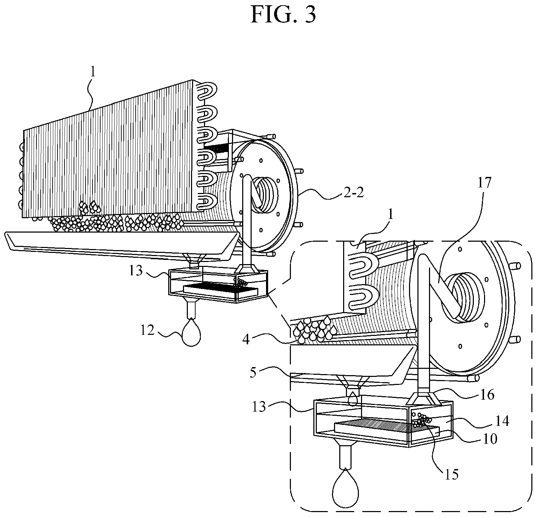

FIG. 3 illustrates an example of air circulation of a low noise cooling device to which a water bucket including an electrolysis electrode at an outlet of the water holder is applied.

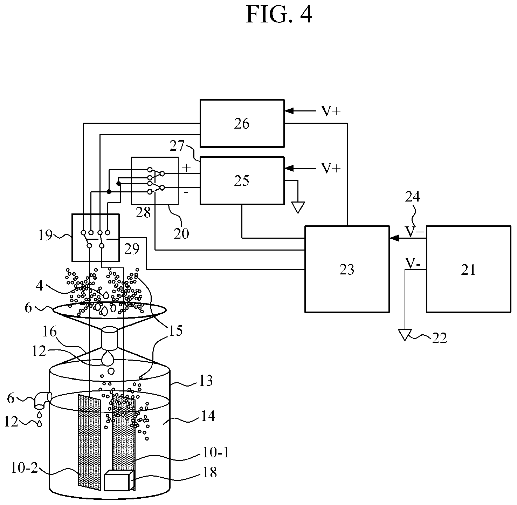

FIG. 4 illustrates a detailed view showing electrolysis of an electrolysis electrode which is completely isolated from an electrode of a power source.
FIG. 5 illustrates a measured resistance and voltage waveform of an electrolyte which operates an apparatus according to the present invention.

### [Modes of the Invention]

Hereinafter, exemplary embodiments of the present invention will be described in detail. However, the present invention is not limited to the exemplary embodiments disclosed below and may be implemented in various forms. The following exemplary embodiments are described in order to enable those of ordinary skill in the art to embody and practice the invention.

Hydrogen produced by electrolyzing water 4 which underwent condensation, was frozen 3 at a cooling part (hereinafter, referred to as an evaporator 1) of a cooling apparatus or a dehumidifier, was then melted, and fell from the evaporator is used to kill fungi 8 and bacteria inside the evaporator, and therefore an improvement can be made to prevent a problem of odors being caused by fungi at a vehicle cooling apparatus and residential and office air conditioners and to prevent a respiratory disease.

When the water 4 which underwent condensation 3, was frozen at the evaporator 1 of the cooling apparatus, was then melted, and fell from the evaporator is in contact with an electrolysis electrode 10, electrical resistance of an electrolyte which is a metal constituent dissolved in the water when the water is condensed in the evaporator 1 is detected by a resistance value measurement circuit 26, and then water 4 and 14 is electrolyzed to produce oxygen and hydrogen.

Here, production of ozone (O₃) is prevented by the oxygen and a porous ceramic catalyst 18 in vicinity of the electrolysis electrode 10 so that only oxygen (O) is dissolved in the water, some hydrogen atoms among electrolyzed hydrogen (H) are dissolved in the water, and a hydrogen bubble 15 evaporates and kills the fungi 8 and bacteria having the possibility of propagation, and therefore, pure cooling for supplying fresh and clean cold air 11 from which the fungi 8 is removed using hydrogen after sterilization mixed in the air is possible. That is, even though the water 4 and 14 is electrolyzed, the porous ceramic catalyst 16 preventing the production of ozone (O₃) in conjunction with the oxygen produced in the electrolysis is placed in the vicinity of the electrolysis electrode 10, and therefore, the production of ozone may be prevented during the electrolysis of water and hydrogen is produced.

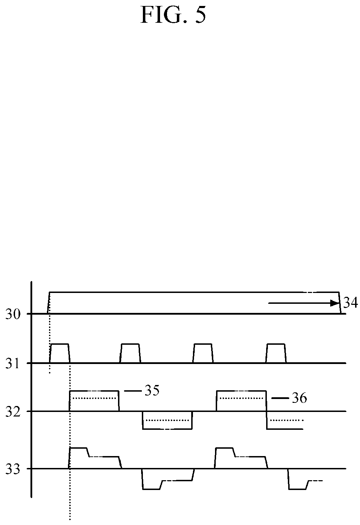

When water vapor in the air is in contact with metal of the evaporator 1, undergoes condensation 3 and is frozen, resistance values of a product of the metal in contact with the water and the water 4 and 14 having a concentration of an electrolyte changed by dust in the air may be measured. When the resistance value of the water is measured by the resistance value measuring circuit 26 for measuring a resistance value of water that is changed according to a concentration of an electrolyte, the resistance is converted into a voltage 27 which is completely isolated 25 from a voltage 24 of a power source 21 according to the resistance value, and an isolated power supply 25 is controlled by a microcontroller 23 so that the voltage 27 is applied to electrolysis electrodes 10-1 and 10-2, as shown in FIG. 5.

Because a negative electrode of a commercialized battery 21 for a vehicle, which is used in most vehicles, is connected to a chassis of a vehicle, electrolysis with a ground 24 of the chassis of the vehicle is prevented only when the voltage 27 completely isolated from the power source 21 of the vehicle is generated and applied to the electrolysis electrodes 10-1 and 10-2 to produce hydrogen.

Therefore, the voltage 27 which is completely isolated from the voltage 24 of the power device 21 is applied to the platinum-plated titanium electrolysis electrodes 10-1 and 10-2, and the power supply 25 which is isolated such that a metal part of the evaporator 1 and the electrolysis electrodes 10-1 and 10-2 are not electrochemically reacted by electrolysis is operated.

In addition, a 2-circuit and 2-contact point relay 20 or a solid state relay is configured to change a direction of an electrical current to the electrolysis electrodes 10-1 and 10-2 at a certain time interval (about 10 seconds), a control signal 28 is controlled by the microcontroller 23 so that changes 32 and 22 of the direction of the electrical current are made at the certain time interval, and therefore an electrochemical reaction of the platinum-plated titanium electrodes is prevented.

Besides the above-described cooling apparatus for vehicles and indoor spaces, a hydrogen generating sterilizer using condensation of a cooling part may be applied to a refrigerator, a dehumidifier, a freezer, an air conditioner, and a water manufacturing device to prevent propagation of bacteria and fungi.

The electrolysis electrodes 10 are installed inside a water holder 5 for receiving water drops 4 of water which underwent condensation 3, was frozen at the evaporator 1 of the cooling apparatus, was then melted, and fell. When the water 4 and 14 is in contact with the electrodes, an electrical resistance of the water is measured 26, and then the current 27 of the power source 25 which is completely isolated from the voltage of the power source flows to the electrolysis electrodes 10 to electrolyze the water 4 and 14, and thus generation 15 of hydrogen occurs, which kills fungi and supplies hydrogen to air so that fresh, clean, and healthy cooling is realized.

In addition, when a water bucket 13 including the electrolysis electrodes 10 is installed under a funnel 6 of the water holder 5 to which water, which underwent condensation 3, was frozen at the evaporator 1 of the cooling apparatus, was melted, and fell therefrom, and the electrical resistance of the water 4 and 14 in contact with the electrolysis electrodes 10 is measured, the current 27 which is completely isolated from the voltage of the power source flows to the electrolysis electrodes 10 and electrolyzes the water to generate hydrogen. When the vaporized hydrogen is transferred to an entrance of a wingless blower 2-2 through a pipe 17, mixed air in which hydrogen and air are mixed is discharged through disks of the wingless blower using centrifugal force, fungi which may occur at the evaporator 1 are killed, and the pure cool air 11 mixed with hydrogen is discharged.

In addition, when indoor air of a cage in which chickens, pigs, and cows are kept, such as a cattle shed, a henhouse, a pigsty, and the like, a plant factory, and a greenhouse to which the GAP process is applied is sterilized using a hydrogen dehumidifying and humidifying apparatus, a hydrogen air conditioner, and a hydrogen cooling apparatus using a technique of spraying hydrogen electrolyzed from water into the air, an infectious disease, avian influenza, and foot-and-mouth disease of animals and plants can be prevented, and odors caused by excretion can be prevented, and therefore animals can be healthily raised.

Furthermore, when indoor air of a food processing and manufacturing factory, a pharmaceutical factory, and a food distribution and sales store that follow the HACCP standard and GMP process is sterilized using hydrogen electrolyzed from water, a hydrogen dehumidifying and humidifying apparatus, a hydrogen air conditioner, and a hydrogen cooling apparatus, and then processed food, manufactured food, and medicine are processed, manufactured, distributed, and sold, spoilage of the food and medicine is prevented. Therefore, shelf life can be extended as much as possible with electrolyzed hydrogen and a method of using the same rather than a conventional method of time temperature tolerance (TTT), which is a method of using a temperature tolerance of storage and distribution.

As described above, hydrogen is supplied to air while killing fungi, and water 12 into which hydrogen is dissolved is drinkable because the water is sterilized with the hydrogen, and therefore the apparatus according to the present invention may be applied to a water manufacturing device.

Therefore, prior to supplying ground water drawn by a pump to a water tank or supplying received rainwater to the water tank, when a water bucket including an electrolysis electrode is installed and an electrical resistance of water in contact with the electrode is measured, a current completely isolated from a voltage of a power source flows to the electrode according to salinity of ground water and an electrical resistance of an electrolyte, and the water is electrolyzed to generate hydrogen. Therefore, a water manufacturing device for killing fungi and bacteria by dissolving hydrogen into water and manufacturing drinkable water can be realized.

## Claims

1. A sterilizing apparatus configured to electrolyze water condensed at cooling parts or evaporators of the cooling apparatus or the dehumidifier or the freezer or the refrigerator to generate hydrogen and sterilize fungi and bacteria in vicinity of the evaporators, wherein the sterilizing apparatus includes:
an electrolysis electrode configured to perform electrolysis when the condensed water is in contact therewith, and at which a porous ceramic catalyst which combines with oxygen generated during the electrolysis is placed to prevent generation of ozone while the water is electrolyzed;
a resistance value measurement part configured to measure a resistance value of the water that changes according to a concentration of an electrolyte dissolved in the water;
a controller configured to adjust an application time by varying a voltage according to the resistance value measured by the resistance value measurement part and control a power supply;
the power supply provided to be isolated such that the voltage isolated from a power device is applied to the electrolysis electrode, which has platinum-plated titanium, to prevent a metal part of the evaporator and the electrolysis electrode from being electrochemically electrolyzed; and
a relay part configured to be controlled to apply the voltage to the electrolysis electrode while changing a direction of an electrical current at a certain time interval.

2. A sterilizing apparatus in which an electrolysis electrode is installed inside a funnel of a water holder into which water condensed and frozen at an evaporator of a cooling apparatus falls, wherein an electrical resistance of the water is measured when the water is in contact with the electrode, a current which is completely isolated from a voltage of a power source flows to the electrode to electrolyze the water and generate hydrogen, and thus fungi is killed and the hydrogen is supplied to air.

3. A sterilizing apparatus in which a water bucket including an electrolysis electrode is installed under a funnel of a water holder into which water condensed and frozen at an evaporator of a cooling apparatus falls, wherein, when an electrical resistance of water in contact with the electrode is measured, a current which is completely isolated from a voltage of a power source flows to the electrode to electrolyze the water and generate hydrogen, and thus fungi is killed and the hydrogen is supplied to air.

4. A cooling apparatus or a dehumidifier or a humidifier or a heat pump or an air conditioner in which hydrogen generated by a sterilizing apparatus of any one of claims 1 to 3 is transferred to an inlet of a wingless blower configured to blow air mixed with the hydrogen to a cooling part or an evaporator with a primary noise, which is a low noise of 40 dB or less, to sterilize the cooling part of the evaporator and blow wind mixed with the hydrogen into an indoor space.

5. A good agricultural practice (GAP) process system configured to sterilize indoor air of a cattle shed, a henhouse, a pigsty, a plant factory, and a greenhouse using hydrogen generated by a sterilizing apparatus of any one of claims 1 to 4 to prevent animals from contracting avian influenza and foot-and-mouth disease and prevent odors caused by excretion.

6. A system configured to sterilize indoor air of a food processing and manufacturing factory, a pharmaceutical factory, and a food distribution and sales store that follow the Hazard Analysis and Critical Control Points (HACCP) standard and a good manufacturing practice (GMP) process using hydrogen generated by a sterilizing apparatus of any one of claims 1 to 4 to prevent spoilage of food and medicine.

7. A drinkable water manufacturing apparatus has a sterilizing apparatus in which condensed water, and rainwater and ground water in addition to the condensed water are filtered by a filter prior to the rainwater and ground water being supplied to a water tank, and, when a water bucket including an electrolysis electrode is installed and an electrical resistance according to salinity or an electrolyte of water which is in contact with the electrolysis electrode is measured, a current which is completely isolated from a voltage of a power source flows to the electrode, and then the water is electrolyzed to generate hydrogen and the hydrogen is dissolved in the water to kill fungi and bacteria.
